# EUROPEAN PATENT APPLICATION

(11) **EP 4 796 025 A2**
(43) Date of publication of application: **26.08.2026**
(21) Application number: 26184140.7
(22) Date of filing: 18.01.2022
(51) Int. Cl.: A61B 5/02

(54) **SYSTEM FOR CHARACTERIZATION OF AN OBJECT AT AN INTRAVASCULAR LOCATION**

(30) Priority: 20.01.2021 EP 21152557
(62) Document number(s) of the earlier application(s) in accordance with Art. 76 EPC: 22702884.2 / 4 280 944
(71) Applicant: Koninklijke Philips N.V., 5656 AE Eindhoven (NL)
(72) Inventor: MUELLER, Manfred, 5656AE Eindhoven (NL); LUCASSEN, Gerhardus Wilhelmus, 5656AE Eindhoven (NL)
(74) Representative: Philips Intellectual Property & Standards

(57) **Abstract**

A device and systems are disclosed for characterization of an object at an intravascular location based on detection of reflected light, wherein a detected reflected light intensity is measured at each wavelength or wavelength range of a set. At least one ratio is determined between the detected reflected light intensities, including at least two discrete wavelengths or wavelength ranges. Based on the at least one ratio, a type and/or composition of the object at the intravascular location can be determined. This provides a simple analytic system when compared to a full spectroscopic analysis.

## Description

### FIELD OF THE INVENTION

This invention relates to a system for classifying an object, such as types of blood clot.

### BACKGROUND OF THE INVENTION

A blood clot in an artery that supplies the brain can lead to ischemic stroke. This is the third commonest cause of death in the developed world and the leading cause of acquired neurological disability. In high-income countries, substantial increases in the absolute numbers of individuals affected by stroke are projected due to increasing life-expectancy, even if stroke incidence rates are reduced or maintained at current levels. Stroke is also a substantial contributor to healthcare costs.

Recent studies have shown the superiority of thrombectomy (mechanical clot removal) over thrombolysis (dissolving the clot) in treating acute strokes, leading to a boom in thrombectomy devices. There are already a significant number of thrombectomy devices widely available, with new thrombectomy devices regularly being introduced onto the market.

A complicating factor for thrombectomy is that blood clots have different compositions depending on clot etiology and age. Some clots are very rich in red blood cells, and others are very rich in fibrin and others have a mixed composition. Depending on the clot composition, blood clots have different mechanical properties that pose different challenges and risks during thrombectomy. Clots rich in red blood cells (so called 'red clots') are fragile with a risk of clot break-up Clots rich in fibrin and/or platelets (so called 'white clots') have an elastic consistency and are difficult to grab. To make matters even more complicated, clots can be inhomogeneous, with composition varying over the length of the clot. This is especially common for larger clots.

The interventional neuroradiologist would prefer to know the blood clot composition ahead of making a thrombectomy device selection. Unfortunately, imaging modalities do not provide sufficient information on the clot composition. In fact it is often difficult to even determine the size of the clot since common imaging modalities like angiography only show the proximal location of the clot but not the distal location of the clot.

Selecting the correct device the first time is critical in thrombectomy, because the window for treatment completion is short. Choosing a suboptimal treatment strategy can necessitate additional clot removal attempts, lengthening the procedure. Each subsequent attempt becomes more difficult as the clot gets compacted. There is also a pronounced first-pass-effect in thrombectomy, and it has been reported that the likelihood of a good clinical outcome nearly doubles if clot removal is achieved during the first pass.

Choosing a different device during the procedure increases costs substantially and choosing the wrong device may increase complication rate. Currently in about a third of procedures, the type of thrombectomy device is switched during the procedure after failure of the primary approach.

The applicant has considered the use of a Diffuse Reflectance Spectroscopy (DRS) system for blood clot discrimination. In diffuse reflectance spectroscopy, white light is used to illuminate a diffuse reflecting sample, like biological tissue. Inside the sample the light is scattered and absorbed. A part of the back-scattered light is collected and analyzed with a spectrometer yielding a spectrum that is characteristic for the sample. Typically, these spectra show a scattering background punctured by characteristic dips caused by absorbers such as blood, water and fat.

A detailed description of the method including ways to analyze the data are given in the following references:
R. Nachabé, B. H. W. Hendriks, A. E. Desjardins, M. van der Voort, M. B. van der Mark, "Estimation of lipid and water concentrations in scattering media with diffuse optical spectroscopy from 900 to 1600 nm", J. of Biomed. Opt. 15 (2010);
R. Nachabé et al., "Estimation of biological chromophores using diffuse optical spectroscopy: benefit of extending the UV-VIS wavelength range to include 1000 to 1600 nm", Biomed. Opt. Express 1(5): p. 1432-1442 (2010); and
R. Nachabé, et al., "Diagnosis of breast cancer using diffuse optical spectroscopy from 500 to 1600 nm: comparison of classification methods" J. Biomed. Opt. 16(8): p. 087010 (2011).

The applicant has demonstrated that a DRS system can measure the composition of artificial blood clots, in ex-vivo and in-vivo settings. However, a full DRS setup is suitable for research but it is too expensive, too heavy and too cumbersome for a commercial product to be used in clinical practice. The spectrometers alone can weigh multiple kilograms and are very expensive for a broad use in clinical practice. The white light source that couples light into an optical fiber is another heavy and expensive component.

To make blood clot discrimination into a product that can be used in daily clinical practice requires a system that is compact, light-weight, robust and cheap.

### SUMMARY OF THE INVENTION

The invention is defined by the claims.

According to examples in accordance with an aspect of the invention, there is provided a detection system, comprising:
an optical fiber arrangement for transmitting light to and from an intravascular location within a subject;
a light source arrangement for generating light with a set of at least two discrete wavelengths or wavelength ranges, wherein the light source arrangement is coupled to the optical fiber arrangement;
a controller for controlling the light source arrangement;
a light detector for detecting reflected light received from the intravascular location via the optical fiber arrangement; and
a processor configured to:
   measure a detected reflected light intensity at each wavelength or wavelength range of the set;
   determine a ratio between the detected reflected light intensities, including said at least two discrete wavelengths or wavelength ranges; and
   from at least the ratio, determine a type and/or composition of object at the intravascular location.

This system is able to detect composition of an object or classify the object based on the detected composition, such as type of tissue or type of blood clot, by determining a ratio between two or more reflected light intensities at particular wavelengths. The term "reflected" is intended to be broad enough to encompass backscattered or fluorescent light. This approach simplifies the system compared to a full spectroscopic analysis. This invention is based on the recognition that absolute measured intensities in the spectra are not very meaningful, so the system can instead use relative intensities between intensities measured for different wavelengths. In particular, absolute intensities vary due to factors that are very difficult to control (like the contact pressure with the tissue, the bending radius of the optical fiber, etc.) and this makes finding patterns in the spectra very difficult. Furthermore, it is not necessary to measure the whole spectra - measuring relative intensities at a few (two or more, for example between four and nine) carefully chosen wavelengths is sufficient. In particular, the various data points in the spectrum are highly correlated so that it is sufficient to process a small number of key wavelengths to discriminate between different types of spectra.

The ratio may be a simple ratio between two reflected intensities, but it may be more complex. For example, the numerator of the ratio may be a combination of intensities (e.g. a sum or difference) and/or the denominator of the ratio may be a combination of intensities (e.g. a sum or difference).

The system for example further comprises an output for outputting the result of determining the object type (e.g. clot type) or the classification of the object to a display device. The display device may be part of the system or a remote device with which the system communicates.

The system may also output a recommendation for the type of treatment to be used, based on the clot type. Different thrombectomy devices can be selected based on the classification of the object, e.g. in the case of white clots and red clots, for example.

The optical fiber arrangement may be a multi-mode fiber, with a single core or with multiple fiber cores. The detector is for example a photodiode or a set of photodiodes. The controller for example comprises electronics to rapidly turn on/turn off light sources of the light source arrangement or modulate such light sources.

The processor is for example configured to determine a type of blood clot. The system may thus be used to assist in selecting a type of treatment for removing a blood clot, such as a type of thrombectomy treatment.

The set of wavelengths for example comprises a first wavelength or wavelength range, which includes a wavelength below 620nm and a second wavelength or wavelength range which includes a wavelength above 620nm.

It has been found that white clots and red clots have different reflection intensities when the reflections at wavelengths above and below 620nm are compared, for example 700nm and 600nm. In particular, white clots show a decreasing intensity while red clots show an increasing intensity across such a range. The light source arrangement for example comprises a set of light sources.

The set for example comprises discrete wavelengths each with a wavelength bandwidth of less than 10nm (FWHM) preferably less than 5 nm. Thus, narrowband light sources may be used or an accurately tunable light source may be used, to make measurements at specific desired wavelengths.

The light source arrangement for example comprises a set of laser diodes.

The set may comprise between four and nine different wavelengths or wavelength ranges. As a minimum, there may be only two wavelengths, but a larger number of wavelengths allows multiple ratios to be determined or else more complex comparisons between reflected light intensities at different wavelengths.

The optical fiber is for example part of a disposable, intravascular part, and the light source arrangement, controller, light detector and processor are part of a console unit. Thus, the higher cost parts can be reused and the part which makes contact with the patient's body is disposable.

The system may further comprise:
an optical unit to multiplex the light of the set of different wavelengths or wavelength ranges into the optical fiber arrangement; and
a beam splitter or fiber splitter for splitting the reflected light and directing it to the detector.

Thus, the optical fiber arrangement carries the combination of the wavelengths from the light source arrangement to the measurement location and also the reflected light from the measurement location, which is split off for detection. Wavelength-selective elements may also be used to separate the various light wavelengths before reaching associated light detectors. These elements are for example color-filters, wavelength-selective mirrors or a prism.

The controller may in one example be configured to operate the light sources in a time division multiplexing manner. In this case, the different wavelengths are delivered to the intravascular location at different times, and this means a single broadband photodetector may be used for all wavelengths.

The controller may in another example be configured to operate the light sources at the same time, but use the inherent different wavelengths to distinguish between the reflected signals. In this case, the different wavelengths are delivered to the intravascular location simultaneously, but the reflected light can be de-multiplexed by using a wavelength-selective element. There is then preferably a different photodetector for each different wavelength or wavelength range.

In yet another example, the controller may be configured to operate the light sources in a frequency division multiplexing manner, whereby each light source is modulated at a unique modulation frequency. In this case, the different wavelengths may again be delivered to the intravascular location simultaneously, but a single broadband photodetector may still be used for all wavelengths because the various wavelengths contributions can be distinguished by their different modulation frequencies.

In one particular example, the light source arrangement comprises light sources with wavelength peaks in the following ranges: $532 nm \pm 10 nm ;$ $642 nm \pm 10 nm ;$ $830 nm \pm 10 nm ;$ and $915 nm \pm 10 nm$

Various different ratios between the reflected light intensities at these different frequencies for example enables classification of a red clot, a white clot, a mixed clot, a vessel wall, or blood. In some of the embodiments the received reflected light may be with slight frequency shift with respect to the wavelength or wavelength range the light source arrangement provides.

The invention also provides a method of classification of object types or determination of the composition of the object, comprising:
receiving light intensity information for a set of at least two discrete wavelengths or wavelength ranges, wherein the light intensity information is related to reflected light from an object at an intravascular location;
ascertaining respective light intensities for each discrete wavelength or wavelength range of the set;
calculating a ratio based on the ascertained light intensities; and
associating a type and/or composition of the object based on the ratio.
This is the processing method applied to the received reflected light.

The method for example further comprises suggesting treatment options based on the determined type or composition. For example, different thrombectomy devices can be selected in the case of white clots, red clots and mixed clots, for example.

The method may also comprise outputting the result of determining the object's type and/or composition to a display device.
The method may comprise, before receiving light intensity information:
generating light with at least two discrete wavelengths or wavelength ranges;
transmitting the light to an intravascular location within a subject;
detecting light at a set of at least two discrete wavelengths or wavelength ranges comprising light reflected from the object at the intravascular location.

This method is then the more complete analysis method rather than just the processing method for processing the detected intensities.

The invention also provides a computer program comprising computer program code means which is adapted, when said program is run on a computer, to implement the processing method defined above.

The invention also provides a processor having stored therein the computer program.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows raw reflection spectra for five different types of tissue;
Fig. 2 shows the spectra scaled by dividing them by the intensity at a common wavelength;
Fig. 3 shows the multiple curves from Fig. 2 scaled and averaged by tissue type;
Fig. 4 shows a decision tree node to discriminate between red and white clots based on this intensity ratio;
Fig. 5 shows a first example of a tissue detection system;
Fig. 6 shows a plot of intensity versus time for a time division multiple approach;
Fig. 7 shows an example of a decision algorithm in the form of a decision tree;
Fig. 8 shows a plot of intensity versus time for a frequency division multiplexing approach based on frequency modulated intensities;
Fig. 9 shows a second example of a tissue detection system;
Fig. 10 shows all possible wavelengths ratios and how well they perform for discriminating between white clots and the vessel wall;
Fig. 11 shows another plot of wavelength ratios to classify between white clots and mixed clots; and
Fig. 12 shows another plot of wavelength ratios to classify between red clots and blood.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a detection system, which is based on the detection of reflected light from an intravascular location, with a detected reflected light intensity measured at each wavelength or wavelength range of a set. At least one ratio is determined between the detected reflected light intensities, including at least two discrete wavelengths or wavelength ranges. From the at least one ratio, a type and/or composition of an object, such as tissue, vessel wall and blood clots, can be determined from an intravascular location. This provides a simple analytic system when compared to a full spectrum analysis.

This invention is based on two insights. First, that absolute intensities in the reflection spectra are not very meaningful, whereas relative intensities between different wavelengths convey more information. Second, it is not necessary to measure the whole spectra - measuring relative intensities at a few carefully chosen wavelengths is sufficient.

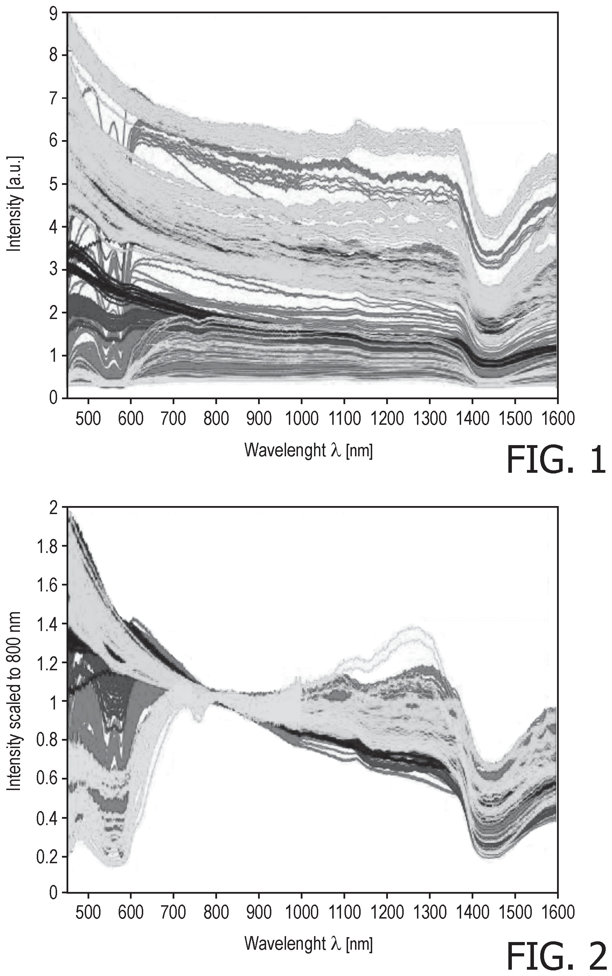

Fig. 1 shows raw reflection spectra (intensity versus wavelength) for five different types of objects (blood, mixed clot, white clot, red clot and vessel wall) and for multiple samples of each tissue type. The different object types are in different shades of grey. The absolute intensities measured vary widely due to factors that are very difficult to control (like the contact pressure with the tissue, the bending radius of the fiber, etc.) and that makes finding patterns in the spectra very difficult.

Fig. 2 shows the spectra scaled by dividing each plot by its respective intensity at a common wavelength λ (here as an example λ = 800 nm). Common features for the different types of objects (e.g. different types of blood clots and vessel wall tissue) become much more visible. It also becomes obvious that not all the data points in the spectra are required for classification of the composition of the objects, since the various data points in the spectra are highly correlated. In fact it is sufficient to look at a small number of key wavelengths to discriminate between different types of spectra.

However, it is not sufficient to look at absolute intensities at these key wavelengths, rather it is necessary to look at relative intensities or intensity ratios. A typical intensity ratio r is given by $r = \frac{I \left({λ}_{1}\right)}{I \left({λ}_{2}\right)}$ where I is the intensity λ₁ and λ₂ are wavelengths or wavelength ranges. Other expressions for relative intensities are possible. For example, in some cases a third wavelength I(λ₃) may be taken into account: $r ′ = \frac{I \left({λ}_{1}\right) - I \left({λ}_{2}\right)}{I \left({λ}_{3}\right)}$

This is of course a subtraction between two simple ratios.

Thus, a ratio may be derived between reflection intensities at a pair of wavelengths, or else multiple such ratios may be derived, or else more complex ratios may be defined with a sum or difference in the numerator and/or the denominator, or more complex functions may be used.

Typically, the value is dimensionless so that it corresponds to a ratio between intensities (or even other similar functions of intensity).

An example will now be presented of how a simple ratio can discriminate between tissue types. A basic example is to decide whether a given spectra is from a red-blood-cell-rich clot ("red clot") or from a fibrin-and/or-platelet-rich clot ("white clot").

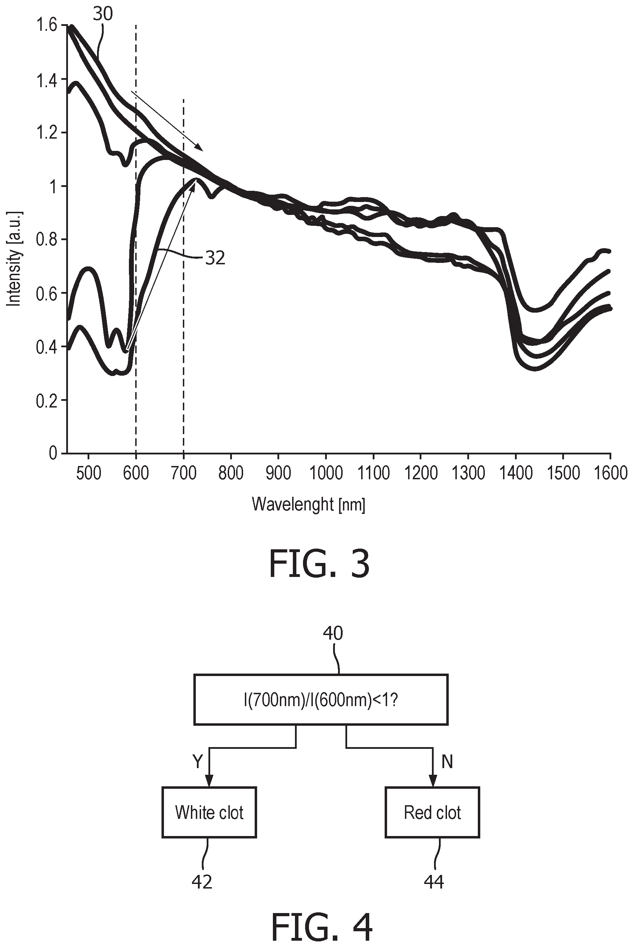

Fig. 3 shows the multiple curves from Fig. 2 scaled and averaged by tissue type. Plot 30 is the curve associated to white clot and plot 32 is associated to the red clot.

The average slope between λ₂=700 nm and λ₁=600 nm for red clots and white clots is indicated by arrows. It can be seen that red and white clots are very distinct at wavelengths below 700 nm. For example, Figs 1 and 2 can be analyzed to show that white clots have spectra with decreasing intensity between 600 nm and 700 nm, while red clots have spectra with increasing intensity in this range. This is visible most clearly in the averages shown in Fig. 3.

Thus, a value: ${r}_{WR} = \frac{I \left(700 nm\right)}{I \left(600 nm\right)}$ can be used to discriminate between the spectra of red and white clots. If r_{WR} < 1 the spectra is likely to be from a white clot, otherwise it is likely to be from a red clot.

Fig. 4 shows a decision tree node to discriminate between red and white clots based on this intensity ratio.

In step 40, the dimensionless ratio between the intensity at 700nm to the intensity at 600nm is derived. A value less than 1 in step 42 indicates a negative slope hence a white clot and a value of 1 or greater indicates a flat or positive slope hence a red clot.

The particular choice of values of λ₁ and λ₂ is not unique. λ₁=680 nm and λ₂=550 nm or any other wavelength combination in the same spectral area may be used. For example a first wavelength or wavelength range may include a wavelength below 620nm and a second wavelength or wavelength range may include a wavelength above 620nm.

Also, λ₁ and λ₂ need not be perfectly monochrome. However, if λ₁ or λ₂ become too broad (for example with a wavelength spread of more than 10 nm) the discrimination will suffer. The choice for a certain wavelength is ideally made taking both discrimination power as well the availability of a suitable light source into account. For that reason, laser diodes are preferred as light sources, as they offer narrow spectral linewidths, high light intensities, a large variety of wavelengths to choose from and easy modulation at a low cost. Current LEDs have typically too broad (>20 nm) emission spectra to be suitable. Furthermore, coupling light into small fiber cores is more efficient with monochromatic laser light.

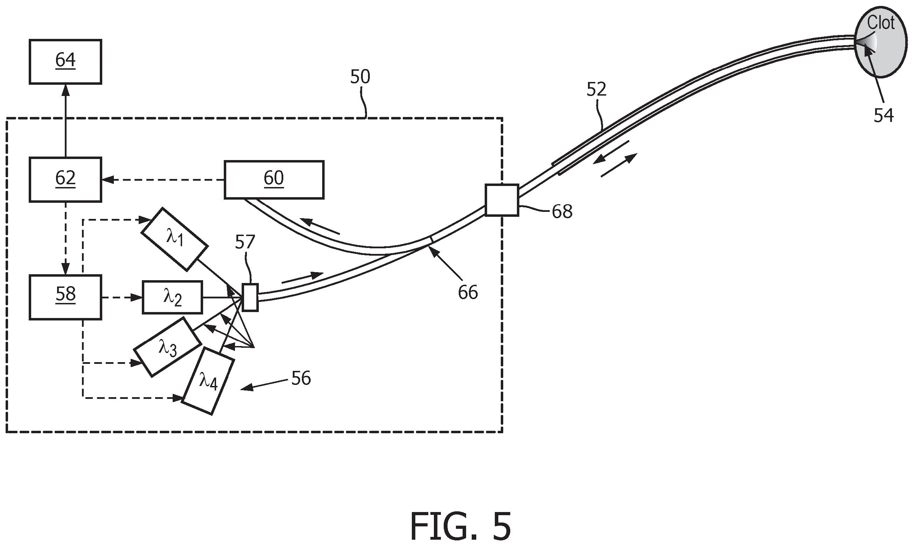

Fig. 5 shows a first example of a tissue detection system. The system comprises a console unit 50 and an optical fiber arrangement 52 for transmitting light to and form an intravascular location 54 within a subject.

The optical fiber arrangement 52 is part of a disposable, intravascular part suitable for insertion into a blood vessel, such as guidewire, catheter or sheath. The optical fiber arrangement 52 for example is a multi-mode optical fiber, with a single core or with multiple fiber cores. It transports light to and from the selected location in the vasculature of the patient.

The console 50 is a re-usable part not for insertion into the patient. The console comprises a light source arrangement 56 for generating light with a set of at least two discrete wavelengths or wavelength ranges. There is for example a set of light sources having 4 to 9 monochrome light sources. The light output from the light source arrangement 56 is coupled to the optical fiber arrangement 52. An optical unit 57 multiplexes the light from the various light sources into the optical fiber guiding it to the intravascular location.

In this example, the light source arrangement comprises discrete separate light sources, such as laser diodes each with their own wavelength.

The laser diodes are controlled by a controller 58 including modulation electronics, or by the processor 62. This is able to rapidly turn on/turn off or modulate the light sources.

A light detector 60 is provided for detecting reflected light received from the intravascular location via the optical fiber arrangement 52.

For the returned reflected light, there is a beam splitter or fiber splitter 66 for directing light to the detector 60. Optionally, wavelength-selective elements (e.g. color-filters, wavelength-selective mirrors or a prism) are used to separate the various light wavelengths before the light detector 60.

A processor 62 implements the analysis, such as the method of Fig. 4. Thus, it measures a detected reflected light intensity at each wavelength or wavelength range of the set of wavelengths and determines at least one ratio between the detected reflected light intensities, including at least two discrete wavelengths or wavelength ranges.

The output 64 from the processor is a type and/or composition of tissue at the intravascular location, in particular in contact with the end of the optical fiber arrangement.

This output is for example provided to a display, where the type and/or composition is displayed to a user. The display may be part of the console unit 50 or it may be a remote device to which the information about the type and/or composition is sent, e.g. wirelessly. The processor 62 may also generate a recommendation of the type of treatment that is appropriate for the identified type and/or composition of the tissue, e.g. clot.

An optical connector 68 enables the disposable intravascular part to be separated from the re-usable console 50. Ideally this connector allows rapid disconnection and reconnection of the intravascular part to allow for easy navigation, torquing etc. of the intravascular part.

In an example based on four light sources, the four wavelengths may be 532 nm, 642 nm, 830 nm and 915 nm, although of course other combinations (and additional wavelengths are possible). Laser diodes with these wavelengths are widely available commercially.

The light beams from the laser diodes can be coupled into the same optical fiber using any of the optical multiplexing techniques known in the art e.g. face coupling from free space, fiber splitters, tapered fibers, dielectric mirrors, beam splitters, holographic gratings, fiber Bragg gratings etc.

The light is transported via one arm of the fiber/beam splitter 66 into the optical fiber arrangement 52. Different wavelengths can be guided via different cores of the same fiber or all wavelengths may be guided in the same core. The fiber directs the light into the tissue to be investigated and collects some of the light that is reflected back from the tissue and guides it back to the console.

Typically, measures will be taken to minimize back reflection from inside the fiber (that is from light that has not been reflected from the tissue). This can be achieved for example by polishing the fiber facet at an angle greater than 10° so that light reflected from the tip falls outside of the acceptance angle of the fiber.

The light coming back from the tissue is guided into the second leg of the fiber/beam splitter 66 and routed to the detector 60. The detector determines the reflected light intensity at all of the wavelengths, I(λ₁),I(λ₂), I(λ₃), I(λ₄).

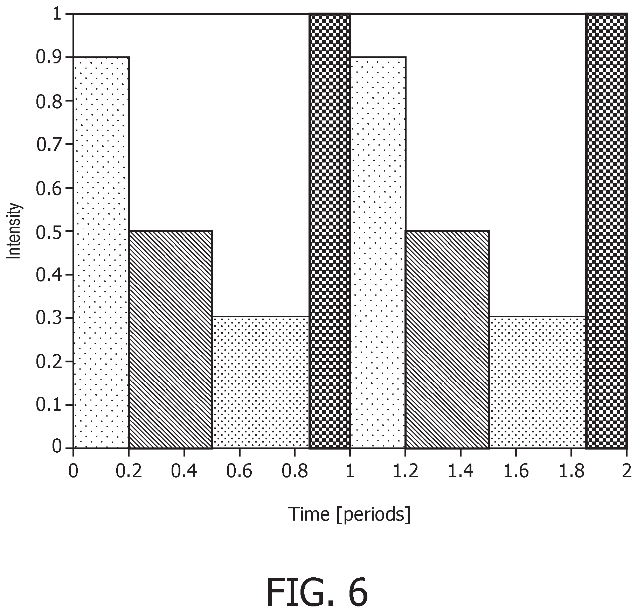

In one approach, to save cost and complexity, the photo detector (e.g. a photo diode or a photo transistor) does not need the capability to discriminate between light of different wavelengths. Instead, to determine I(λ₁ )... I(λ₄ ), this approach uses a time-division driving scheme for the lasers as shown in Fig. 6.

It is to be noted that in an alternative embodiment of the system, the disposable device comprises the elements 56, 57, 60, 66 in a proximal portion of the disposable device, which remains outside the patient's body, and one or more optical connector elements 68 enable the disposable intravascular device to be connected to the re-usable console 50. Additionally, the console may have an electrical connector to the proximal portion of the disposable device, for transmission/reception of electrical signals (e.g. control of the light source arrangement and detector located in the proximal portion, for example in a handle).

Fig. 6 shows a plot of intensity versus time for a time division multiplexing approach. The four different hatch types relate to the four different wavelengths or wavelength ranges. In this driving scheme, the lasers are modulated such a way that at most one laser diode is emitting at any point in time.

The light intensity detected by the photodiode during the time when only the laser with wavelength λ₁ is active will be used to determine I(λ₁), the light intensity detected by the photodiode during the time when only the laser with wavelength λ₂ is active will be used to determine I(λ₂) and so on. The fraction of time that each laser is emitting does not need to be equal but can be chosen to compensate for example for different light intensities or for different absorption in the tissue.

The measured intensities I(λ₁) to I(λ₄) are used by the processing unit to determine the type and/or composition of the tissue, that the light was reflected from. To do this the processing unit first determines the intensity ratios according to equations (1) or (2) above.

For this example with four wavelengths, I(λ₁)... I(λ₄), there are six distinct ratios according to equation (1): I(λ₁)/I(λ₂), I(λ₁)/1(λ₃), I(λ₁)/I(λ₄), I(λ₂)/I(λ₃), I(λ₂)/I(λ₄), I(λ₃)/I(λ₄). Other combinations are not distinct, e.g. I(λ₂)/I(λ₁) is simply the inverse of I(λ₁)/I(λ₂).

Many additional different ratios may be derived using equation (2).

Based on the intensity ratios, the processing unit then determines the type and/or composition of the tissue. The output classification result (e.g. 'red clot') or the composition information (e.g. '30% red blood cells') is made visible/audible to the physician either through a user interface (UI) of the system or coded by different LEDs on the system or connector. This classification can be done using any combination of decision methods, e.g. decision trees, support vector machines (SVMs), nearest neighbor algorithms, or similar machine learning and classification/regression methods.

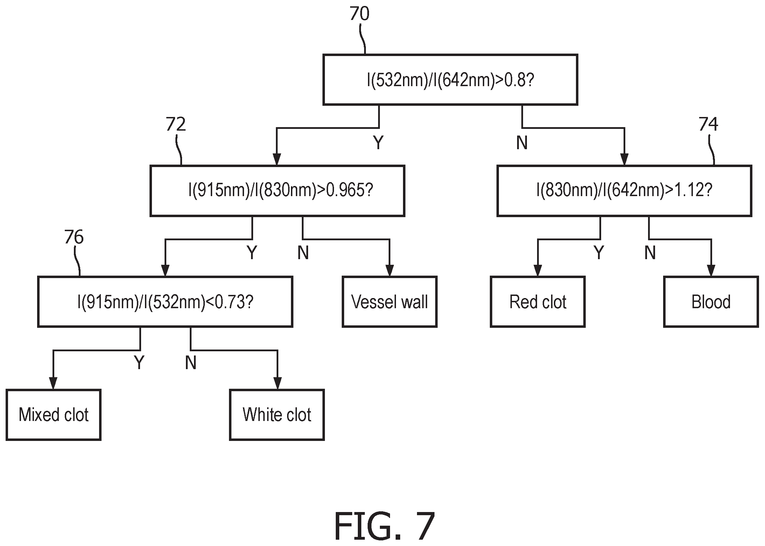

An example of a decision algorithm in the form of a decision tree is depicted in Fig. 7. The basic decision tree has been trained using half of the spectra from Fig. 1 and validated on the other half yielding an overall accuracy of 85%.

In Fig. 7, the ratio I(532nm)/I(642nm) is compared with 0.8 in step 70, and depending on the result, a further comparison is made of either (i) I(915nm)/I(830nm) with threshold 0.965 in step 72 or (ii) I(830nm)/I(642nm) with threshold 1.12 in step 74.

Depending on the result of step 72, a fourth comparison is made of I(915nm)/I(532nm) with a threshold of 0.73 in step 76.

These four ratios enable the system to distinguish between a red clot, a white clot, a mixed clot, a vessel wall and blood, as shown.

Adding additional decision nodes to the tree can improve accuracy further. For example adding three additional decision nodes to the tree (with five different wavelengths) can enable discrimination between all five classes with even greater overall accuracy.

These sensitivity and specificity values achieved using the method of Fig. 7 are already sufficient for clinical use and can be further improved by more sophisticated algorithms and/or by using more wavelengths (as mentioned above).

In the example above, the various laser diodes are turned on and off to provide time division multiplexing. Instead, the laser diodes can be periodically modulated at different modulation frequencies.

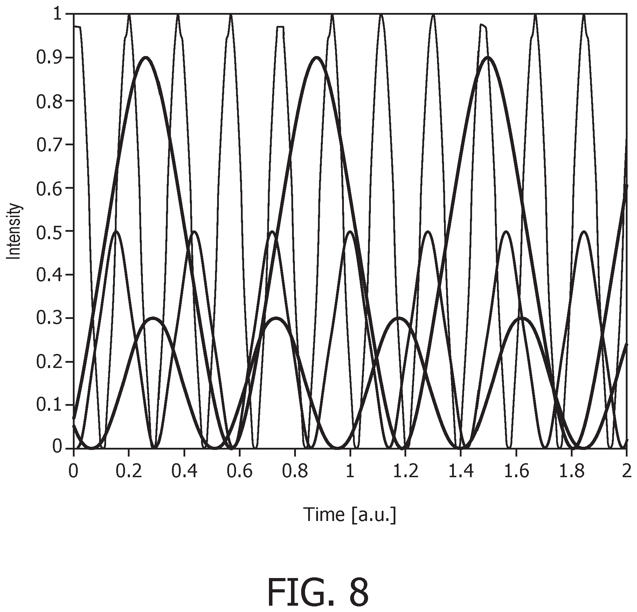

Such a frequency division multiplex system is shown in Fig. 8. Four overlapping intensity plots are shown. Each light intensity waveform has a different modulation frequency (as well of course as a different wavelength of the light itself). There are various well-known techniques to extract the individual frequency components from such a mixed signal, e.g. (fast) Fourier transformation techniques, multiplying the modulation signal with the measured signal and integrating over multiple periods, etc.

Instead of using a single photo detector for all wavelengths together, a separate photo detector may be used for each wavelength.

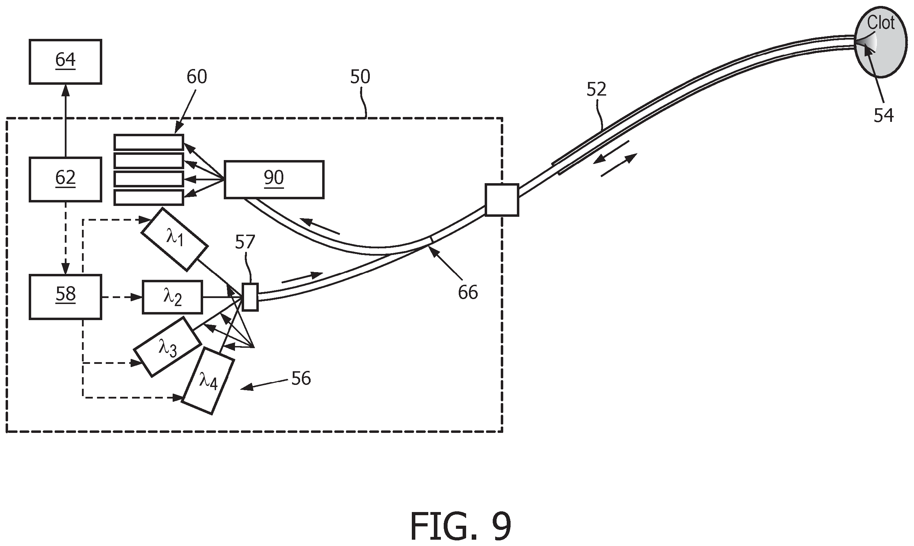

A system with separate photo detectors is shown in Fig. 9. It is similar to Fig. 5 but has a wavelength selective component 90 to make sure that each photo detector (four are shown together forming the overall detector 60) only measures the signal from its intended wavelength. In the simplest case, this wavelength-selective element can be a color filter in front of each detector. Other possibilities include prisms, wavelength selective mirrors, gratings, multivariate optical element with filter characteristics of the principal spectral components of the blood clot types (e.g. red, mixed white), etc.

This wavelength selectivity of the detectors can be combined with the time division multiplex or frequency multiplex driving schemes from the examples above. For example, it is possible to explicitly detect signals at a wavelength different from the emission frequency, which may occur if the tissue is fluorescent.

The use of multiple laser diodes is described above, but there may instead be one or more multi-wavelength source(s) or a tunable light source. Integrated optics may also be used, integrated with the light source arrangement into a single chip. LEDs may also be used, for example with color filters or with other wavelength selective elements to reduce the wavelength spread. However, laser diodes provide the most suitable current technology based on cost and performance.

Decision tree examples are shown above for classifying the clot types and vessel wall. However, other approaches may be used, for example include analysis using machine learning algorithms.

Different ratios of intensities may be of interest in discriminating between different tissue types, as is already clear from Fig. 7.

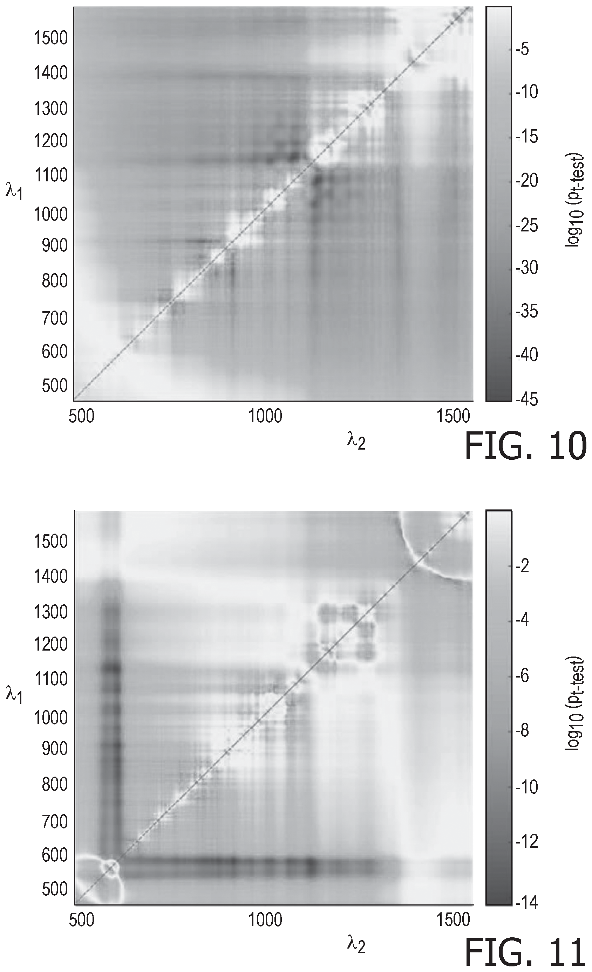

For example, Fig. 10 shows all possible wavelengths ratios and how well they perform in discriminating between white clots and the vessel wall. The darker the grey scale level, the better the discrimination. The best combinations are in this case are in rather narrow bands involving either 915 nm or 1154 nm.

Fig. 11 shows another example of the analysis that classifies between white clots and mixed clots. In this case, the best discrimination uses a wavelength around 580 nm or around 535 nm.

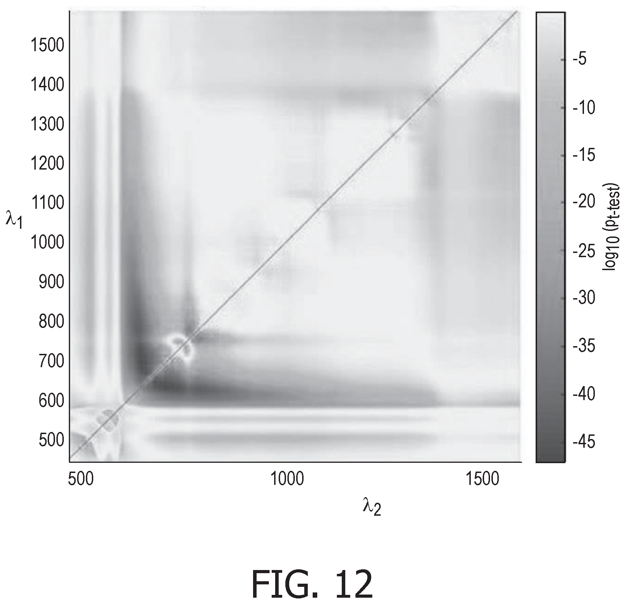

Fig. 12 shows another example of the analysis that classifies between red clots and blood. In this case, the best discrimination uses a wavelength around 640 nm or alternatively around 830 nm.

Thus different ratios may be used to distinguish between different tissue types.

The invention is of particular interest for the treatment of ischemic stroke. Apart from stroke the invention may however also find application in analyzing clots or occlusions in other vessels, such as the coronaries, the lung vessels and the peripheral vessels in the leg.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims, such as for example that of the controller. The processor may be substituted by multiple processors or processing units, thereby dividing various functionalities of a single processor.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A device for characterization of an object, the device comprising a processor (62) configured to:
receive light intensity information for a set of at least two discrete wavelengths or wavelength ranges, wherein the light intensity information is related to reflected light from an object at an intravascular location;
ascertain respective light intensities for each discrete wavelength or wavelength range of the set;
calculate a ratio based on the ascertained light intensities; and
associate a type and/or composition of the object based on the ratio.

2. The device of claim 1, wherein the processor (62) is configured to determine a type and/or composition of a blood clot.

3. The device of claim 1 or 2, wherein the processor is further configured to output a recommendation for a treatment to be used, based on the type and/or composition of the object.

4. A system for characterization of an object, comprising:
an optical fiber arrangement (52) for transmitting light to and from an intravascular location within a subject;
a light source arrangement (56) for generating light with at least two discrete wavelengths or wavelength ranges, wherein the light source arrangement is coupled to the optical fiber arrangement;
a light detector for detecting reflected light at a set of at least two discrete wavelengths or wavelength ranges received from the intravascular location via the optical fiber arrangement; and
a device according to any of the claims 1 to 3.

5. The system of claim 4, wherein the set comprises a first wavelength or wavelength range which includes a wavelength below 620nm and a second wavelength or wavelength range which includes a wavelength above 620nm.

6. The system of claim 4 or 5, wherein the set comprises discrete wavelengths each with a wavelength bandwidth of less than 10nm FWHM, for example less than 5nm FWHM.

7. The system of any one of claims 4 to 6, wherein the light source arrangement (56) comprises a set of laser diodes.

8. The system of any one of claims 4 to 7, wherein the set comprises between four and nine different wavelengths or wavelength ranges.

9. The system of any one of claims 4 to 8, wherein the optical fiber arrangement (52) is part of a disposable, and the light source arrangement, light detector and processor are part of a console unit (50).

10. The system of any one of claims 4 to 9, further comprising:
an optical unit (57) to multiplex the light of the set of different wavelengths or wavelength ranges into the optical fiber arrangement; and
a beam splitter or fiber splitter (66) for splitting the reflected light and directing it to the detector.

11. The system of any one of claims 4 to 10, wherein the processor is configured to modulate the light sources to provide time division multiplexing or to modulate the light sources with different modulation frequencies to provide frequency division multiplexing.

12. The system of any one of claims 4 to 11, wherein the processor is configured to operate the light sources simultaneously and a wavelength selective element is used to separate the different wavelengths for detection.

13. The system of any one of claims 4 to 12, wherein the light source arrangement (56) comprises light sources with wavelength peaks in the following ranges: $532 nm \pm 10 nm ;$ $642 nm \pm 10 nm ;$ $830 nm \pm 10 nm ;$ and $915 nm \pm 10 nm .$

14. A method of characterization of an object, comprising:
receiving light intensity information for a set of at least two discrete wavelengths or wavelength ranges, wherein the light intensity information is related to reflected light from an object at an intravascular location;
ascertaining respective light intensities for each discrete wavelength or wavelength range of the set;
calculating a ratio based on the ascertained light intensities; and
associating a type and/or composition of the object based on the ratio.

15. The method of claim 14, comprising, before receiving light intensity information:
generating light with at least two discrete wavelengths or wavelength ranges;
transmitting the light to an intravascular location within a subject;
detecting light at a set of at least two discrete wavelengths or wavelength ranges comprising light reflected from the object at the intravascular location.
